(19) Europäisches Patentamt
European Patent Office
Office européen des brevets

(11) Veröffentlichungsnummer: **0 027 268**
**A2**

(12) # EUROPÄISCHE PATENTANMELDUNG

(21) Anmeldenummer: **80106183.9**

(22) Anmeldetag: **10.10.80**

(51) Int. Cl.³: **C 07 D 513/04**
A 61 K 31/505
//(C07D513/04, 227/00, 239/00)

(30) Priorität: **12.10.79 US 84471**

(43) Veröffentlichungstag der Anmeldung:
**22.04.81 Patentblatt 81/16**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Lemahieu, Ronald Andrew**
**18 Spruce Road**
**North Caldwell, N.J.(US)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80(DE)**

(54) **Chinazolin-Derivate, Verfahren und Zwischenprodukte zu deren Herstellung, Arzneimittel enthaltend solche Chinazolin-Derivate und deren pharmazeutische Verwendung.**

(57) Chinazolin-Derivate der allgemeinen Formel

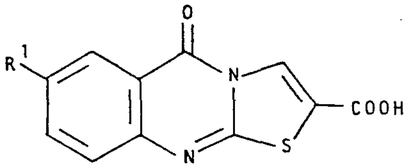

1

worin R¹ Methylthio oder Methylsulfinyl bedeutet, und die pharmazeutisch verwendbaren Salze davon besitzen wertvolle antiallergische Eigenschaften und können demnach zur Behandlung oder Verhütung von allergischen Reaktionen, wie Bronchialasthma, verwendet werden. Die genannten Verbindungen und deren Salze können nach verschiedenen Verfahren hergestellt werden.

EP 0 027 268 A2

Croydon Printing Company Ltd

F.Hoffmann-La Roche & Co.Aktiengesellschaft,Basel,Schweiz

RAN 4050/10

Chinazolin-Derivate, Verfahren und Zwischenprodukte zu deren Herstellung, Arzneimittel enthaltend solche Chinazolin-Derivate und deren pharmazeutische Verwendung.

Die vorliegende Erfindung betrifft Chinazolin-Derivate der allgemeinen Formel

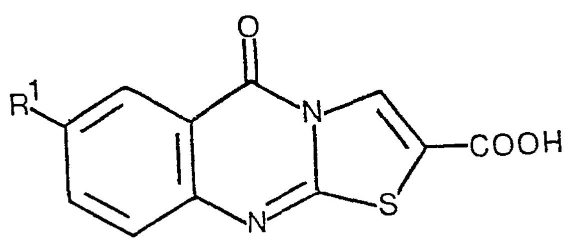

I

worin $R^1$ Methylthio oder Methylsulfinyl bedeutet, sowie pharmazeutisch verwendbare Salze davon.

Der in dieser Beschreibung verwendete Ausdruck "niederes Alkyl" bedeutet Alkylgruppen mit 1-7, vorzugsweise 1-4, Kohlenstoffatomen, wie Methyl, Aethyl, Propyl, Butyl und dergleichen. Der Ausdruck "Halogen", wie nachstehend verwendet, betrifft die vier Formen Fluor, Chlor, Brom und Jod.

7-Methylthio oder Methylsulfinyl-5-oxo-5H-thiazolo-[2,3-b]chinazolin-2-carbonsäure und deren pharmazeutisch

Kbr/3.9.80

verwendbare Salze können erfindungsgemäss so hergestellt werden, dass man

a) ein Anthranilsäure-Derivat der allgemeinen Formel

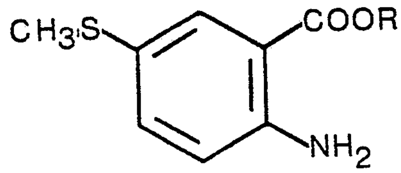

II

worin R Wasserstoff oder niederes Alkyl bedeutet, mit einem 2-Halothiazol der allgemeinen Formel

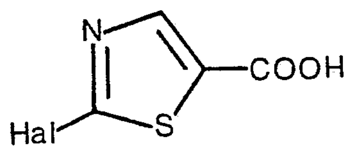

III

kondensiert, oder

b) eine Verbindung der allgemeinen Formel

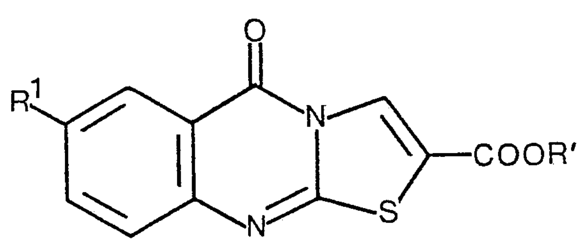

IV

worin R' niederes Alkyl bedeutet und $R^1$ die oben angegebene Bedeutung besitzt, hydrolysiert, oder

c) 7-Methylthio-5-oxo-5H-thiazolo[2,3-b]chinazolin-2-carbonsäure oxidiert, und

d) erwünschtenfalls, eine erhaltene Verbindung in ein pharmazeutisch verwendbares Salz überführt.

Die Kondensation eines Anthranilsäure-Derivates der obigen Formel II mit einem 2-Halothiazol der obigen Formel III wird vorzugsweise in einem Temperaturbereich von etwa 100 bis etwa 180°C durchgeführt. Die Kondensation kann in Gegenwart oder Abwesenheit eines geeigneten Lösungsmittels durchgeführt werden, gewöhnlicherweise ohne Zugabe eines Lösungsmittels. Zweckmässigerweise kann auch ein hochsiedendes, polares Lösungsmittel, wie 2-Methoxyäthanol, Triglyme, Dimethylformamid und dergleichen verwendet werden. Im weiteren kann die Kondensation auch in Gegenwart oder Abwesenheit eines Katalysators durchgeführt werden. Beispiele solcher Katalysatoren sind Alkalimetalljodide, wie Natriumjodid, Lithiumjodid und Kaliumjodid, wobei das Kaliumjodid bevorzugt ist, oder eine geeignete Säure, wie die Ameisensäure und dergleichen.

Die Hydrolyse einer Verbindung der obigen Formel IV zur erwünschten Säure der Formel I kann in einem Ueberschuss eines Alkalimetallhydroxyds, z.B. Natriumhydroxyd, Kaliumhydroxyd und dergleichen, in einem Temperaturbereich von etwa 0 bis etwa 30°C erfolgen. Besonders bevorzugt wird die Reaktion bei etwa 25°C durchgeführt. Der eigentlichen Hydrolyse folgt ein Erhitzen zum Rückfluss in einer organischen Carbonsäure, wie Essigsäure, Propionsäure und dergleichen. Die erwünschte 7-Methylthio oder Methylsulfinyl-5-oxo-5H-thiazolo[2,3-b]chinazolin-2-carbonsäure kann nach üblichen Verfahrensweisen abgetrennt werden, beispielsweise durch Kristallisation und dergleichen.

Die 7-Methylthio-5-oxo-5H-thiazolo[2,3-b]chinazolin-2-carbonsäure kann zur 7-Methylsulfinyl-5-oxo-5H-thiazolo-[2,3-b]chinazolin-2-carbonsäure oxidiert werden. Diese

Oxidation wird unter Verwendung eines Oxidationsmittels, z.B. Natriumperjodat und dergleichen, durchgeführt. Die Temperatur für diese Oxidation ist nicht kritisch, und man arbeitet normalerweise bei Raumtemperatur.

Die Verbindungen der Formel I bilden mit Basen pharmazeutisch verwendbare Salze. Beispiele von Basen sind Alkalimetallhydroxyde, wie Natriumhydroxyd, Kaliumhydroxyd und dergleichen, Erdalkalimetallhydroxyde, wie Calciumhydroxyd, Bariumhydroxyd und dergleichen, Alkalimetallalkoholate, wie Natriumäthanolat, Kaliumäthanolat und dergleichen, organische Basen, wie Piperidin, Diäthylamin, N-Methylglucamin und dergleichen.

Die als Ausgangsmaterial verwendeten Verbindungen der Formeln II und III sind bekannt oder können in an sich bekannter Weise, d.h. in zur Herstellung der bekannten Verbindungen analoger Weise, hergestellt werden. Die als Ausgangsmaterial verwendeten Verbindungen der Formel IV sind neu und ebenfalls Gegenstand vorliegender Erfindung. Sie können nach den oben für die Verfahrensaspekte a) und c) beschriebenen Methoden hergestellt werden, nämlich diejenigen Verbindungen, worin $R^1$ Methylthio bedeutet, durch Kondensation eines Anthranilsäure-Derivates der Formel II mit einem niederen Alkylester eines 2-Halothiazols der Formel III, und diejenigen Verbindungen, worin $R^1$ Methylsulfinyl bedeutet, durch Oxidation des so erhaltenen entsprechenden Methylthio-Derivates. Die niederen Alkylester der 2-Halothiazole der Formel III sind bekannt oder könnnen in an sich bekannter Weise, d.h. in zur Herstellung der bekannten Verbindungen analoger Weise, hergestellt werden.

Die Verbindungen der Formel I, d.h. die 7-Methylthio-5-oxo-5H-thiazolo[2,3-b]chinazolin-2-carbonsäure und die 7-Methylsulfinyl-5-oxo-5H-thiazolo[2,3-b]chinazolin-2-carbonsäure, und deren pharmazeutisch verwendbare Salze hemmen Hautanaphylaxie bei Ratten und sind daher zur Verhütung von allergischen Reaktionen, z.B. zur prophylaktischen Behandlung von Bronchialasthma, verwendbar. Die anti-anaphylaxische Wirksamkeit kann durch den passiven Hautanaphylaxie-Test (PCA-Test) bei Ratten gezeigt werden. Bei diesem Test werden Ratten durch Injektion von Antisera in die Haut passiv lokal sensibilisiert. Nach einer Latenzzeit von 24 Stunden wird die Testverbindung, d.h. im vorliegenden Falle die 7-Methylthio oder Methylsulfinyl-5-oxo-5H-thiazolo[2,3-b]chinazolin-2-carbonsäure, intraperitoneal verabreicht. Nach 5 Minuten wird Reagin sowie Evans Blaustoff intravenös appliziert. Die Fälle, die mit einer lokalisierten Antigen-Antikörperreaktion verbunden sind, führen zur Bildung von Hautschwellungen, deren Grösse gemessen wird. Die Fähigkeit der Testverbindung, die Grösse der Hautschwellung bei den behandelten Tieren im Vergleich zu den Hautschwellungen der unbehandelten Tiere zu verringern, wird als Mass für die Aktivität der Testverbindungen genommen.

Wird die 7-Methylthio-5-oxo-5H-thiazolo[2,3-b]chinazolin-2-carbonsäure, welche einen $DL_{50}$-Wert von 450 mg/kg intraperitoneal aufweist, bei einer Dosis von 16 mg/kg intraperitoneal verabreicht, beträgt die Verminderung der Grösse der Hautschwellungen 80%.

Wird die 7-Methylsulfinyl-5-oxo-5H-thiazolo[2,3-b]-chinazolin-2-carbonsäure in einer Dosis von 16 mg/kg intraperitoneal verabreicht, beträgt die Verminderung der Grösse der Hautschwellungen 50%.

Wird die 7-Methylthio-5-oxo-5H-thiazolo[2,3-b]chinazolin-2-carbonsäure, welche einen $DL_{50}$-Wert von $>$ 1000 mg/kg oral aufweist, im obigen Test in einer oralen Dosis von 32 mg/kg verabreicht, beträgt die Verminderung der Grösse der Hautschwellungen 87%. Die Verbindung weist einen $ID_{50}$-Wert von 0,12 mg/kg nach oraler Verabreichung auf.

Wird die 7-Methylsulfinyl-5-oxo-5H-thiazolo[2,3-b]-chinazolin-2-carbonsäure im obigen Test in einer oralen Dosis von 32 mg/kg verabreicht, beträgt die Verminderung der Grösse der Hautschwellungen 67%. Die Verbindung weist nach oraler Verabreichung einen $ID_{50}$-Wert von 1,82 mg/kg auf.

Die 7-Methylthio-5-oxo-5H-thiazolo[2,3-b]chinazolin-2-carbonsäure, die 7-Methylsulfinyl-5-oxo-5H-thiazolo[2,3-b]-chinazolin-2-carbonsäure oder deren pharmazeutisch verwendbare Salze können oral oder parenteral als antiallergische Mittel, z.B. für die prophylaktische Behandlung von Bronchialasthma, in den individuellen Erfordernissen angepassten Dosen verabreicht werden. Für therapeutische Zwecke können sie beispielsweise oral oder parenteral durch Verabreichen von therapeutischen Mengen in einer üblichen Darreichungsform, wie Tabletten, Kapseln, Elixiere, Suspensionen, Lösungen, Aerosole und dergleichen verwendet werden. Sie können in Mischung mit konventionellen pharmazeutischen Trägerstoffen oder Excipientien, wie z.B. Maisstärke, Calciumstearat, Magnesiumcarbonat, Calciumsilikat, Dicalciumphosphat, Talk, Lactose und dergleichen, verabreicht werden. Weiterhin können sie in Gegenwart von Puffern oder Stoffen zur Einstellung der Isotonizität verabreicht werden. Die pharmazeutischen Dosierungsformen können erwünschtenfalls konventionellen pharmazeutischen Behandlungsweisen unterworfen werden, beispielsweise können sie sterilisiert werden. Wie bereits oben erwähnt, können die Dosierungen den individuellen Bedürfnissen angepasst werden. Darüberhinaus können die Darreichungsformen andere therapeutisch wertvolle Substanzen enthalten.

Die Wirkstoffmenge in den einzelnen oben beschriebenen Darreichungsformen ist variabel. Es wird jedoch bevorzugt, Kapseln oder Tabletten zu verabreichen, die von etwa 10 mg bis etwa 20 mg 7-Methylthio oder Methylsulfinyl-5-oxo-5H-thiazolo[2,3-b]chinazolin-2-carbonsäure oder eine äquivalente Menge eines pharmazeutisch verwendbaren Salzes davon

enthalten.

Die Häufigkeit der Verabreichung der Darreichungsform an den Patienten wird in Abhängigkeit von der Wirkstoffmenge in der Darreichungsform und in Abhängigkeit von den Bedürfnissen des Patienten und sonstigen Gegebenheiten erfolgen. Unter gewöhnlichen Umständen können jedoch täglich bis zu etwa 20 mg/kg Wirkstoff in mehreren Dosen verabreicht werden. Es ist darauf hinzuweisen, dass die oben angegebenen Dosierungen lediglich Beispiele darstellen und somit weder den Umfang noch die praktische Anwendung der vorliegenden Erfindung beschränken.

Die nachfolgenden Beispiele erläutern die Erfindung. Alle Temperaturen sind in °C angegeben.

Beispiel 1

Eine Mischung von 4,45 g Methyl 5-methylthioanthranilat, 4,0 g Methyl 2-chlorthiazol-5-carboxylat und 0,23 g Kaliumjodid wird gut gerührt und in einem Oelbad bei 160° während 40 Minuten erhitzt. Der erhaltene Festkörper wird mit 50 ml einer gesättigten Lösung von Natriumbicarbonat behandelt und mit Chloroform extrahiert. Der über Magnesiumsulfat getrocknete Extrakt wird unter vermindertem Druck zu einem Festkörper eingeengt, welcher mit 100 ml Aether behandelt und abfiltriert wird, wobei man 5,1 g Methyl 7-methylthio-5-oxo-5H-thiazolo[2,3-b]chinazolin-2-carboxylat erhält, Schmelzpunkt 195-197°.

Beispiel 2

5,1 g Methyl 7-methylthio-5-oxo-5H-thiazolo[2,3-b]-chinazolin-2-carboxylat werden durch 16-stündiges Rühren bei Raumtemperatur in 160 ml 1N Natriumhydroxydlösung und 200 ml Methanol hydrolysiert. Nach dem Ansäuern mit Essigsäure wird der grösste Teil des Lösungsmittels unter vermindertem Druck abgedampft. Dann gibt man Wasser zu, filtriert den Festkörper ab, welcher danach in Essigsäure während einer Stunde zum Rückfluss erhitzt wird. Danach lässt man die Lösung abkühlen und filtriert den kristallinen Festkörper ab, wobei man 3,57 g reine 7-Methylthio-5-oxo-5H-thiazolo[2,3-b]chinazolin-2-carbonsäure erhält, Schmelzpunkt 253-255°.

Beispiel 3

Zu einer in einem Eisbad gekühlten Suspension von 1,022 g 7-Methylthio-5-oxo-5H-thiazolo[2,3-b]chinazolin-2-carbonsäure in 25 ml Wasser werden 3,5 ml 1,0N Natriumhydroxydlösung gegeben. Nach wenigen Minuten werden 0,824 g Natriumperjodat zugegeben. Die erhaltene Lösung wird während 2 Stunden in einem Eisbad und danach während 4 Stunden bei Raumtemperatur gerührt. Nach Zugabe von 3,8 ml 1N Salzsäure wird der erhaltene Festkörper abfiltriert und aus Essigsäure umkristallisiert, wobei man 0,829 g reine 7-Methylsulfinyl-5-oxo-5H-thiazolo[2,3-b]chinazolin-2-carbonsäure erhält, Schmelzpunkt 243-244°.

## Beispiel A

Kapseln der nachfolgenden Zusammensetzung werden hergestellt:

| | mg/Kapsel | |
|---|---|---|
| | 10 mg | 20 mg |
| 7-Methylthio-5-oxo-5H-thiazolo[2,3-b]chinazolin-2-carbonsäure | 10 mg | 20 mg |
| Lactose | 215 mg | 205 mg |
| Maisstärke | 60 mg | 60 mg |
| Magnesiumstearat | 3 mg | 3 mg |
| Talk | 12 mg | 12 mg |
| Total | 300 mg | 300 mg |

Verfahren:

Die 7-Methylthio-5-oxo-5H-thiazolo[2,3-b]chinazolin-2-carbonsäure, die Lactose und die Maisstärke werden in einem geeigneten Mischer vermischt, und darauf wird das Ganze durch eine geeignete Mühle gemahlen. Die Mischung wird mit dem Magnesiumstearat und Talk vermischt und auf einer geeigneten Kapselvorrichtung abgefüllt.

Beispiel B

Tabletten der nachfolgenden Zusammensetzung werden hergestellt:

| | mg/Tablette | |
|---|---|---|
| | 10 mg | 20 mg |
| 7-Methylthio-5-oxo-5H-thiazolo[2,3-b]chinazolin-2-carbonsäure | 10 mg | 20 mg |
| Lactose | 182 mg | 172 mg |
| Mikrokristalline Cellulose | 60 mg | 60 mg |
| Modifizierte Stärke | 15 mg | 15 mg |
| Maisstärke | 30 mg | 30 mg |
| Magnesiumstearat | 3 mg | 3 mg |
| Total | 300 mg | 300 mg |

Verfahren:

Die ersten 5 Bestandteile werden in einem geeigneten Mischer vermischt. Dann gibt man das Magnesiumstearat zu, mischt während 5 Minuten und verpresst auf einer geeigneten Tablettenpresse.

## Beispiel C

Tabletten der nachfolgenden Zusammensetzung werden hergestellt:

| | mg/Tablette | |
|---|---|---|
| | 10 mg | 20 mg |
| 7-Methylthio-5-oxo-5H-thiazolo[2,3-b]chinazolin-2-carbonsäure | 10,0 mg | 20,0 mg |
| Lactose | 264,0 mg | 254,0 mg |
| Vorgelatinierte Stärke | 17,5 mg | 17,5 mg |
| Maisstärke | 35,0 mg | 35,0 mg |
| Modifizierte Stärke | 17,5 mg | 17,5 mg |
| Magnesiumstearat | 6,0 mg | 6,0 mg |
| Total | 350,0 mg | 350,0 mg |

Verfahren:

Die ersten vier Bestandteile werden in einem geeigneten Mischer vermischt und mit Wasser granuliert. Das Ganze wird danach durch eine geeignete Mühle gemahlen. Dann mischt man die modifizierte Stärke und das Magnesiumstearat zu und verpresst auf einer geeigneten Presse zu Tabletten.

Patentansprüche

1. Chinazolin-Derivate der allgemeinen Formel

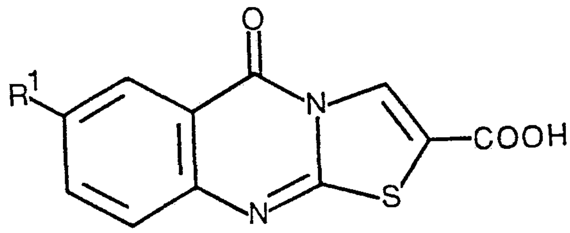

I

worin $R^1$ Methylthio oder Methylsulfinyl bedeutet, und pharmazeutisch verwendbare Salze davon.

2. 7-Methylthio-5-oxo-5H-thiazolo[2,3-b]chinazolin-2-carbonsäure.

3. Verbindungen der allgemeinen Formel

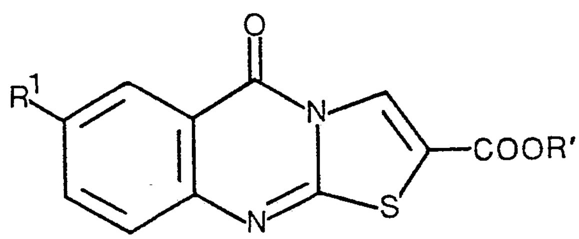

IV

worin R' niederes Alkyl und $R^1$ Methylthio oder Methylsulfinyl bedeuten.

4. Chinazolin-Derivate der in Anspruch 1 angegebenen allgemeinen Formel I und pharmazeutisch verwendbare Salze davon als pharmazeutische Wirkstoffe.

5. Chinazolin-Derivate der in Anspruch 1 angegebenen allgemeinen Formel I und pharmazeutisch verwendbare Salze davon als Antiallergika.

6. Verfahren zur Herstellung von Chinazolin-Derivaten

der in Anspruch 1 angegebenen allgemeinen Formel I und den pharmazeutisch verwendbaren Salzen davon, dadurch gekennzeichnet, dass man

a) ein Anthranilsäure-Derivat der allgemeinen Formel

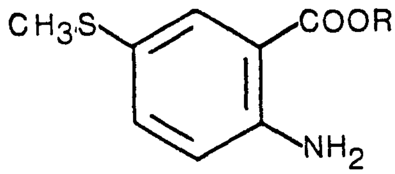

II

worin R Wasserstoff oder niederes Alkyl bedeutet, mit einem 2-Halothiazol der allgemeinen Formel

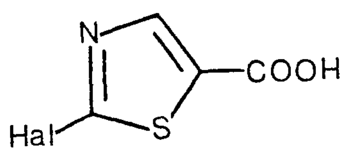

III

kondensiert, oder

b) eine Verbindung der allgemeinen Formel

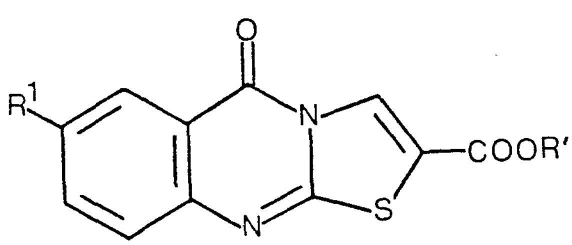

IV

worin R' niederes Alkyl bedeutet und $R^1$ die oben angegebene Bedeutung besitzt,

hydrolysiert, oder

c) 7-Methylthio-5-oxo-5H-thiazolo[2,3-b]chinazolin-2-carbonsäure oxidiert, und

d) erwünschtenfalls, eine erhaltene Verbindung in ein pharmazeutisch verwendbares Salz überführt.

7. Arzneimittel, enthaltend ein Chinazolin-Derivat der in Anspruch 1 angegebenen allgemeinen Formel I oder ein pharmazeutisch verwendbares Salz davon.

8. Antiallergikum, enthaltend ein Chinazolin-Derivat der in Anspruch 1 angegebenen allgemeinen Formel I oder ein pharmazeutisch verwendbares Salz davon.

9. Verwendung von einem Chinazolin-Derivat der in Anspruch 1 angegebenen allgemeinen Formel I oder einem pharmazeutisch verwendbaren Salz davon bei der Bekämpfung bzw. Verhütung von Krankheiten.

10. Verwendung von einem Chinazolin-Derivat der in Anspruch 1 angegebenen allgemeinen Formel I oder einem pharmazeutisch verwendbaren Salz davon bei der Bekämpfung oder Verhütung von allergischen Reaktionen.

Patentansprüche
"für OESTERREICH"

1. Verfahren zur Herstellung von Chinazolin-Derivaten der allgemeinen Formel

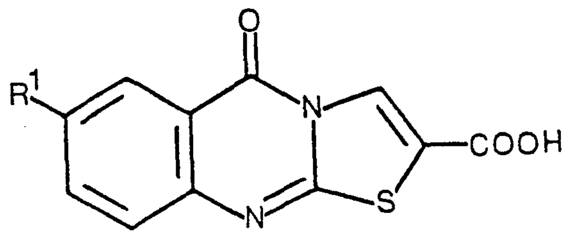

I

worin $R^1$ Methylthio oder Methylsulfinyl bedeutet, und pharmazeutisch verwendbaren Salzen davon, dadurch gekennzeichnet, dass man

a) ein Anthranilsäure-Derivat der allgemeinen Formel

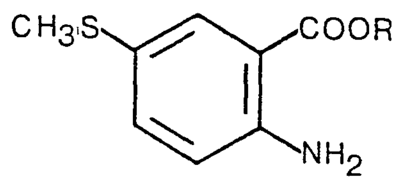

II

worin R Wasserstoff oder niederes Alkyl bedeutet, mit einem 2-Halothiazol der allgemeinen Formel

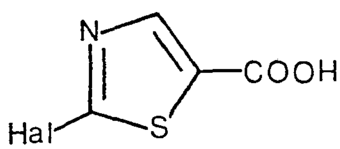

III

kondensiert, oder

b) eine Verbindung der allgemeinen Formel

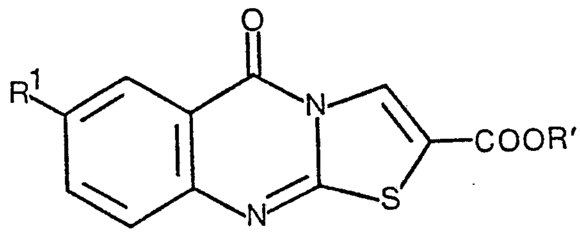

IV

worin R' niederes Alkyl bedeutet und $R^1$ die oben angegebene Bedeutung besitzt,
hydrolysiert, oder

c) 7-Methylthio-5-oxo-5H-thiazolo[2,3-b]chinazolin-2-carbonsäure oxidiert, und

d) erwünschtenfalls, eine erhaltene Verbindung in ein pharmazeutisch verwendbares Salz überführt.

2. Verfahren nach Anspruch 1, dadurch gekennzeichnet, dass man in Verfahrensvariante a) eine Verbindung der Formel II, worin R Wasserstoff oder Methyl bedeutet, und in Verfahrensvariante b) eine Verbindung der Formel IV, worin $R^1$ Methylthio bedeutet, als Ausgangsmaterial verwendet.

3. Verfahren nach Anspruch 1 oder 2, dadurch gekennzeichnet, dass man nach der Verfahrensvariante a) oder b) arbeitet und dass man in Verfahrensvariante b) eine Verbindung der Formel IV als Ausgangsmaterial verwendet, worin $R^1$ Methylthio bedeutet.